# EUROPEAN PATENT APPLICATION

(11) **EP 4 230 215 A1**
(43) Date of publication of application: **23.08.2023**
(21) Application number: 21811405.6
(22) Date of filing: 11.10.2021
(51) Int. Cl.: A61K 36/81, A61P 35/00

(54) **SOLANUM MELONGENAETHANOLIC EXTRACT OF SEEDS OF SOLANUM MELONGENA, METHOD FOR OBTAINING IT, PHARMACEUTICAL COMPOSITION CONTAINING IT AND USE THEREOF AS AN ANTITUMOUR AGENT**

(30) Priority: 13.10.2020 ES 202031034
(71) Applicant: Universidad de Granada, E-18071 Granada (ES); Cellbitec, S.L., 04007 Almería (ES)
(72) Inventor: BERMÚDEZ PÉREZ, Francisco, 04007 Almería (ES); PRADOS SALAZAR, Jose Carlos, 18071 Granada (ES); MELGUIZO ALONSO, Consolación, 18071 Granada (ES); PORRES FOULQUIE, Jesús Mª, 18071 Granada (ES); MESAS HERNÁNDEZ, Cristina, 18071 Granada (ES); MARTÍNEZ MARTÍNEZ, Rosario, 04007 Almería (ES); GALISTEO MOYA, Milagros, 18071 Granada (ES); ORTIZ QUESADA, Raúl, 18071 Granada (ES); CABEZA MONTILLA, Laura, 18071 Granada (ES); LÓPEZ-JURADO ROMERO DE LA CRUZ, María, 18071 Granada (ES)
(74) Representative: Elzaburu S.L.P.
(86) International application number: PCT/ES2021/070738
(87) International publication number: WO 2022/079331

(57) **Abstract**

The present invention provides a method for obtaining an ethanolic extract from mature seeds of *Solanum melongena,* preferably defatted, characterized in that they are extracted with a hydroalcoholic solution containing 50% ethanol, at acid PH, preferably under a nitrogen atmosphere and for short periods of time, no longer than 1-2 h. The extracts obtained from different varieties of *Solanum melongena* have a high polyphenol content and show potent antitumor activity against colon adenocarcinoma, pancreatic adenocarcinoma and glioblastoma multiforme cell lines, even against chemotherapy-resistant lines, but with low toxicity to human hepatocytes. Therefore, the extracts of the invention are proposed for use in the treatment of colon adenocarcinoma, pancreatic adenocarcinoma and glioblastoma multiforme, including those resistant to currently used chemotherapies.

## Description

### Technical field

The present invention relates to the field of biology, botany, nutrition and biomedicine. Specifically, it relates to obtaining an ethanolic extract of vegetable origin to be used as an antitumor agent, from mature seed meal of *Solanum melongena,* optionally defatted.

### Background of the invention

Cancer is defined as a set of related diseases in which certain cellular alterations cause a loss of control of the proliferation process that leads to excessive cell division and the ability of these malignant cells to spread to other tissues. Cancer, in general, has a high incidence in the world population and requires the development of new, more effective treatments, especially in advanced stages of the disease in which patients have a very poor prognosis. Within the field of oncology, colorectal cancer (CRC) is considered a real public health problem due to its current prevalence and incidence projections in the coming years.

According to data from 2018 from the Spanish Society of Medical Oncology (*Sociedad Española de Oncología Médica* or SEOM), CRC ranks third among cancers with the highest incidence in both sexes and worldwide. In Spain, CRC is the most frequently diagnosed cancer (year 2019) in both sexes, being the second most frequent in both men and women. Regarding its mortality rate, CRC was the second leading cause of cancer-related deaths in both sexes as of December 2019, according to the National Institute of Statistics. This mortality has a clear growth trend that has been shown to be related to lifestyle and diet.

Among the risk factors for the development of CRC are: i) age, since it is diagnosed most frequently in patients over 50 years of age (90% of cases) without other pathologies or predisposing diseases; ii) dietary factors, which play an essential role and are under continuous investigation; in fact, excessive alcohol consumption, overweight and obesity, and certain types of food (processed meat) have been linked to this pathology; iii) predisposing diseases, especially the presence of intestinal polyps or inflammatory bowel disease, which means that these patients should be considered at high risk for the development of CRC; iv) a history of CRC; v) so-called genetic or family factors, given that in 25% of cases there is a family history and in 10% a hereditary component, and vi) lastly, lifestyle, with physical inactivity being an important factor.

Despite the fact that the treatment and prevention of CRC have undergone great advances in recent years, the results, in terms of cure or reduction in incidence, are far from satisfactory. We must emphasize that, in the most advanced phase of the disease, in which patients develop distant metastases (especially in the liver), the results of the different applied therapies tested are especially poor. These results are dramatically reflected in the prognosis of these patients, who have a very low survival rate. The currently approved treatments for CRC range from surgery, when the tumor is resectable, to the use of chemotherapy based on different cytotoxics such as oxaliplatin, irinotecan, 5-fluorouracil, capecitabine, Utefos, TAS-102, Raltitrexed (either alone or in combination) or radiotherapy. In recent years, new biological drugs active against CRC have been developed, including the monoclonal antibodies cetuximab, panitumumab, bevacizumab and a recombinant fusion protein (aflibercept) that has very specific therapeutic indications (Loree et al., 2017; Nappi et al., 2018; Bregni et al., 2020). Nonetheless, and as the mean survival of these patients clearly indicates (15 to 20.5 months), the results are very limited. Therefore, improving the prognosis of these patients and improving their survival requires the development of new strategies that add therapeutic activity to preventive action (ldrees et al., 2019; Reglero et al., 2019).

The development of new therapeutic and prevention strategies covers a large number of research fields that range from nanotechnology, gene therapy, immunotherapy using strategies that activate the immune system or the use of new compounds or extracts of plant or animal origin, which may be active in the treatment of CRC or adjuvant to current therapies to improve the response to treatment. In this context, the study of the effect of plant extracts or derivatives on the viability, proliferation and survival of tumor cells has gained great interest in recent years (Goyal et al., 2017). In fact, this line of research has been endorsed and supported by the National Cancer Institute (USA) since 1960, the year in which the antitumor and preventive activity of different plant extracts against certain carcinomas began to be evaluated (Huang et al., 2013). Plants in general, and their extracts in particular, have numerous applications in medicine since they are i) a direct source of therapeutic agents, ii) a raw material for the manufacture of more complex semi-synthetic drugs, iii) for providing the chemical structures of their active principles, which can serve as a model for the preparation of synthetic drugs and iv) for the use of said principles as taxonomic markers in the search for new drugs. These possible applications are due to the phytochemicals present in the plants and their extracts, of which more than 5,000 have been identified in seeds, fruits, roots, tubers, leaves, etc., among which are phenolic compounds, carotenoids, vitamins, alkaloids, terpenes and terpenoids, nitrogenous compounds and organosulfur compounds (Thapliyal et al., 2018).

Although they have been extracted from plants or parts thereof, the possible activity of many phytochemical compounds has been studied individually, after being isolated or, at most, by analyzing the activity of fractions obtained in chromatographic processes in which their presence has been demonstrated. Thus, for example, Akihisa T. et al. (2011) isolated salannin from the seeds of the *Azadirachhta indica* tree and demonstrated that salannin and certain other limonoids exhibit a potent inhibitory effect on the melanogenesis process. To isolate these compounds, they carried out an extraction by reflux for 3 hours with n-hexane in order to subsequently isolate the limonoids present by chromatography. Salannin also possesses antitumor activity in neuroblastoma and osteosarcoma cell lines (Cohen et al., 1996) with an IC₅₀ of 133 ± 6 µM in the NIE.115 neuroblastoma cell line and 89 ± 12 µM in the 143B.TK osteosarcoma cell line.

Moreover, Kaempferol 3-sophorotrioside is related to antioxidant and hepatoprotective activity (Toshiyuki et al., 2001).

Ting et al. (2017) developed extracts containing myricomplanoside. For this purpose, they produced an ethanolic extract (95%) from the seeds of *Astragalus chinensis* in order to subsequently make fractions and obtain some compounds isolated by chromatography. These fractions, one of which contained myricomplanoside, exhibit an antioxidant, anti-inflammatory and antiproliferative activity.

In turn, the group of capsianosides is characterized by interfering with the function of the cytoskeleton by modulating the reorganization of actin filaments (Hashimoto et al., 1997).

Phenolic compounds, in general, have attracted the interest of the scientific community due to their great structural diversity as well as their wide bioactivity. These phenolic compounds hold essential functions in the reproduction and growth of plants, act as defense mechanisms against pathogens, parasites and predators, and are also responsible for giving plants their color. They are not only beneficial for plants, but also play an important role in human health, as antioxidant, anticancer, antibacterial, and anti-inflammatory agents (Huang et al., 2013; Bonta et al., 2019). Due to their bioactivity, there are numerous drugs and patents whose main components are polyphenols, that is, compounds that have more than one phenolic group per molecule. This is the case of Neumentix^{™}, (https://www.kemin.com/na/en-us/products/neumentix), a patented supplement (US patent 9839661), obtained by Kemin, (represented in Spain by Univar) and obtained from harvested green mint (KU 10 and KI42 ranges) after a drying process. This supplement, rich in polyphenols of the rosmarinic, salvianolic and caftaric acid types, has been shown, through clinical trials, to provide great benefits in cognitive performance. In addition, the polyphenols that characterize this supplement act as antioxidant agents, reducing oxidative stress, promoting neuronal growth and protecting nerve cells in the brain. Similarly, polyphenols found in grapes, blueberries, and other fruits and vegetables have been studied for their ability to lower the risk of developing neurodegenerative diseases. In addition, they have been used as a supplement to minimize the effects of age, especially memory loss, as is the case with Optimized Curcumin with Neurophenol^{™} (mixture of blueberry and grape extracts), from Douglas Laboratories^{®} (Valencia, Spain: https://www.douglaslabs.es/). There are also commercialized polyphenolic extracts patented from the grape seed (Vitaflavan^{®}, product of DRT - Les Derivés Résiniques et Terpéniques, Dax, France: https://www.vitaflavan.com/es/), from the pomace of the red grape (Eminol^{®}) and red wine (Provinols^{®}, from Sucren/Vitimed, distributed by Seppic, La Garenne Colombes, France: https://www.seppic.com/provinoistm-0) that have antioxidant properties.

More than 25% of the drugs used during the last 20 years are directly derived from plants, while another 25% are derived from modified natural products (Amin et al., 2009). It is worth mentioning that only between 5% and 15% of the plants used for medicinal purposes have been subject to research aimed at obtaining their bioactive compounds. This highlights the importance of the search for new drugs from plant species (Rivas-Morales et al., 2016; Wong et al., 2018).

The genus *Solanum* (Solanaceae) has the most species (more than 1500 registered species) of any genus in the Solanaceae family, of which the potato (*Solanum tuberosum*), the tomato (*Solanum lycopersicum*) and the eggplant (*Solanum melongena*) deserve to be highlighted due to their economic and cultural importance. Because the species of the genus *Solanum* contain various phytochemicals such as alkaloids, this genus has been used since ancient times for medicinal purposes. The alkaloids that these species present have been of great interest due to their broad biological activity, such as antimicrobial, antirheumatic, antioxidant, as well as anticancer (Jayakumar et al., 2016).

The plants of the species *Solanum melongena* (eggplant) seem to originate in Southeast Asia, with their main center of origin possibly being in India, a country where they have been cultivated since ancient times (at least since 2000 BC). Their ancestral cultivation in Africa and China is also known; this last country may have been a center of secondary origin in which varieties with small fruits were developed. Eggplant cultivation spread throughout North Africa, from where its cultivation seems to have spread to Muslim Spain and other warm Mediterranean countries, such as France, Italy and Greece. The numerous current varieties that have been developed coexist with ancestral varieties of Asian origin and varieties of other species, the fruit of which is also called eggplant, and with which they are closely related, such as *Solanum aethiopicum,* a species including varieties of ancestral domestication in sub-Saharan Africa.

*Solanum melongena* is one of the plant species for which therapeutic applications are known due to the research into and identification of its different bioactive compounds starting from different parts of the plant.

Thus, for example, document JP2002226387A relates to a pharmaceutical composition that contains dried eggplant powder used to topically treat wounds, burns, acne, athlete's foot, stomatitis, inflammation, tumors, etc.

Some other studies, such as those by Zhao Dong-Yin et al. (2020), describe the isolation and identification of various sesquiterpenoids and other compounds from the sepals of *Solanum melongena* by ethanolic extraction (70%) by reflux, followed by the isolation of the bioactive compounds by petroleum ether. These compounds were tested *in vitro* against cervical-uterine cancer, adenocarcinoma and gastric cancer cell lines.

Studies have also been carried out using the roots in which different compounds have been isolated that were tested *in vitro* in breast cancer lines. In this case, the method carried out for the development of the extract is through a reflux process with 70% ethanol and fractionation. In the studies by Yin Xin et al. in 2019, several sesquiterpenoids were isolated and none of those tested showed cytotoxicity against breast cancer (MCF-7), liver cancer (HepG2) or cervical-uterine cancer (HeLa) cell lines. Yang Bing-You's 2020 studies describe the isolation of terpenes, lignans and other compounds also from roots, some of which showed moderate cytotoxicity against the same cell lines.

As in the sepals and roots, bioactive compounds (glycoalkaloids, and, in particular, solamargine) have also been found in the skin of the fruit that have been tested, and have shown effectiveness against liver carcinoma (Fekry et al., 2019) and lung cancer (Shen et al., 2017), and which were obtained by methanolic and ethanolic extraction (70%) and using HPLC (70%). Finally, anthocyanins and delphinidin derivatives from eggplant skin, which were isolated by extraction carried out by immersion in methanol, have been shown to have antioxidant activity in colon cancer cell lines (Jing et al., 2015).

The anticancer activity of glycoalkaloids has already been observed by Lee et al. in 2004, who carried out a study on different glycoalkaloids from potatoes, eggplants and tomatoes and their activity on HT-29 colon cancer cells and HepG2 liver cancer cells. The major glycoalkaloids identified in *Solanum melongena* were solamargine and solanine. In general, the glycoalkaloids were shown to have some anticancer activity, although their effectiveness was greater in trials with the liver cancer cell line than against colon cancer cells.

Thus, although some studies analyze the presence of bioactive compounds in different parts of the plant of the *Solanum melongena* species and analyze, individually for each compound, its activity in different types of tumors, there is little research in relation to the molecular mechanisms by which these compounds act, studies that would be essential to elucidate the pathways by which these functional extracts, or isolated compounds, could act against tumor cells. Among the few works that address aspects of the molecular activity of the extracts, there is the study carried out by Nishina et al. (2015), wherein a bioactive compound, dioscin, was isolated from a methanolic extract from *Solanum melongena* L., which acts as an inhibitor of melanogenesis by deregulating the phospho-CREB protein, which binds and activates MITF (Microphthalmia-associated transcription factor involved in the development of melanocytes and osteoclasts).

Furthermore, although there are studies focused on other parts of the plant, the seeds seem to have received less attention: there are no recently published articles that study the antitumor activity of functional extracts from *Solanum melongena* seeds or isolate bioactive compounds therefrom. Only one study published in 1985 describes the isolation of 3 saponins from *Solanum melongena* seeds by refluxing with methanol for 4 hours at 65 ºC and extract concentration under reduced pressure. However, this study did not report antiproliferative activity of these compounds (Kintia and Shvets, 1985).

Given the need to develop alternative therapeutic strategies against CRC, it would be interesting if new anticancer agents could be found that act thereon, preferably with selective antitumor activity. For simplicity purposes, it would be interesting if said activity could be found in a plant extract, preferably from a plant with edible parts, such as the eggplant, whose extract exhibits such an activity, without the need to isolate the bioactive compounds present therein (even though said isolation is possible), and if the extract could be obtained by means of a methodology that would facilitate its use as an anticancer agent at concentrations in which said extract would show reduced toxicity against non-cancer cells. It would also be a preferable additional feature if said extract contained more than one component that exhibited anticancer activity (or therapeutic activity in general) per se, which would allow the preparation of pharmaceutical compositions combined with two or more of said bioactive compounds. In addition, it would be helpful to know, preferably in some depth, the molecular and cellular mechanisms by which said extracts cause tumor cell death.

The present invention provides a solution to this problem, which also include several of the above-mentioned desired additional advantages.

### Description of the invention

In one aspect, the invention relates to a method for obtaining an ethanolic plant extract from mature seeds of *Solanum melongena,* which comprises the steps of:
a) grinding the seeds to obtain meal;
b) extracting the meal from step a) using a cold extraction hydroalcoholic solution at acid pH; and
c) optionally, defatting the mature seeds by mechanical cold pressing before carrying out steps a) and b).

Preferably, the method of the invention is carried out under the following conditions:
a) the seed is ground to obtain meal with a particle size between 100 µm and 150 µm; and/or
b) the meal obtained in step a) is extracted with the hydroalcoholic extraction solution under the following conditions:
   i. temperature equal to 4 ºC,
   ii. in a nitrogen-reducing atmosphere,
   iii. the extraction solution is made up of ethanol, bidistilled water and 12N hydrochloric acid in proportions 50:50: 0.2 by volume,
   iv. pH equal to 2,
   v. the mixture of the meal and the extraction solution is kept under stirring for 30 minutes after having reached conditions i to iv, and where the ethanolic extract is obtained by centrifuging the mixture of the extraction solution and the meal and collecting the supernatant.

It is very specifically preferred that the defatting be carried out prior to obtaining the meal, at a temperature between 40 and 50 ºC and with an extraction speed of 2 to 3 kg of seeds/hour.

In another preferred embodiment, a new extraction is carried out on the residue resulting from the first extraction, specifically on the precipitate resulting from obtaining an initial extract after centrifugation, applying the following sub-steps:
i) the precipitate resulting from centrifuging the mixture of meal and extraction solution is resuspended in the extraction solution,
ii) the obtained suspension is again stirred for 30 minutes under the conditions of step b) defined in claim 2,
iii) the suspension is subjected to centrifuging, collecting the supernatant, and
iv) the ethanolic extract results from mixing the supernatant obtained in iii) with the first supernatant obtained.

The method of the invention may include an additional final step in which the ethanol from the ethanolic extract obtained is partially or totally evaporated.

In another aspect, the invention relates to an ethanolic extract from mature *Solanum melongena* seeds, particularly with a high polyphenol content. Preferably, said extract will be an extract obtainable by the method of the present invention.

The ethanolic extract, according to the tests of Example 1, has a total polyphenol content ranging between 11.16 and 27.59 µg gallic acid equivalents/mg extract. Preferably, the total polyphenol content ranges from 12.06 to 27.59 µg gallic acid equivalents/mg extract (which are the extreme values determined from extracts from defatted meal). The case in which the content of total polyphenols ranges between 11.16 and 18.41 and/or the reducing capacity ranges between 5.33 and 6.31 µg gallic acid equivalents/mg extract (values obtained in extracts of variety S0506) is also of interest.

In another possible embodiment, compatible with the previous ones, the ethanolic extract comprises at least salannin and/or capsianoside II. Preferably, in addition to the two previous compounds, the ethanolic extract comprises at least one more bioactive compound selected from the group of kaempferol 3-sophorotrioside, myricomplanoside and aryllatose B, or combinations of these three compounds, with particular preference that the ethanolic extract comprise, in addition to salannin and capsianoside, myricomplanoside and/or aryllatose B, or aryllatose B and kaempferol 3-sophorotrioside. More preferably, the ethanolic extract comprises at least the five previous compounds: salannin, capsianoside II, myricomplanoside, aryllatose B and kaempferol 3-sophorotrioside.

As in the case of the method of the invention, and in the other aspects of the invention that are described below, the embodiments that correspond to extracts obtained from meal of mature seeds of *Solanum melongena* varieties, preferably defatted, are preferred. Thus, in the case of the ethanolic extract of the invention, it is preferred that it meet the definition based on the biochemical parameters obtainable from mature seeds of *Solanum melongena* varieties, that is, the extract where:
i) the content of total polyphenols varies from 12.06 to 27.59 µg gallic acid equivalents/mg extract, and/or
ii) the extract comprises at least salannin, capsianoside II and myricomplanoside.

The extracts of the invention obtained from mature seeds of sample S0506 are also preferred, both from defatted and non-defatted meal, so those where:
i) the total polyphenol content ranges from 11.16 to 18.41 and/or the reducing capacity ranges from 5.33 to 6.31 µg gallic acid equivalents/mg extract, and/or
ii) the extract comprises salannin, capsianoside II, myricomplanoside, aryllatose B and kaempferol 3-sophorotrioside.

In any of the definitions, it is preferred that the extract have been obtained by the method of the present invention, in its most general definition, or in any of its possible embodiments; included within these embodiments is the possibility of carrying out the optional final additional step in which the final, partial or total, evaporation of the ethanol is carried out. It is preferred, again, that the step of defatting the mature seeds by cold mechanical pressing has been carried out before carrying out step a) of grinding the seed and step b) of extracting the meal obtained and, especially, that each of the possible steps (defatting, grinding and extracting per se) are carried out with the defining characteristics expressed when describing the possible embodiments of the method of the invention, including carrying out a second extraction on the precipitate resulting from centrifugation that gives rise to the initial extract.

A pharmaceutical composition is also an aspect of the present invention, which will be considered a pharmaceutical composition of the present invention which comprises an extract of the present invention in its formulation, in any of the possible embodiments that have been described above, such an example being that of the extracts obtained by the method of the present invention in which the final, partial or total, evaporation of ethanol has been carried out. The composition may be a combined pharmaceutical composition that comprises at least one anticancer agent in addition to those present in the extract. In another possible embodiment, also compatible with any other embodiment, the composition may also comprise one or more pharmaceutically acceptable excipients and/or carriers.

It can also be considered that the above aspect of the invention implies that the use of an extract of the present invention for the preparation of a pharmaceutical composition is also included within the present invention, particularly if it is intended for the treatment of cancer, especially if it is selected from the group of colorectal cancer, pancreatic cancer and glioblastoma.

An extract of the present invention, or a pharmaceutical composition of the present invention, for use in the treatment of a type of cancer which is selected from the group of colorectal cancer, pancreatic cancer and glioblastoma are also aspects of the invention. More specifically, the cancer may be selected from the group of colon adenocarcinoma, pancreatic adenocarcinoma, and glioblastoma multiforme. From aspects of the invention, the preferred ones are both those referring to the extract and to the pharmaceutical composition, the extracts obtained from mature seeds, especially defatted, but also without defatting, and the pharmaceutical compositions prepared therefrom.

The aspects related to the therapeutic use of an extract of the invention or of a pharmaceutical composition of the invention can also be defined, or are related to, a method of treatment of a subject suffering from a cancer selected from colorectal cancer, pancreatic cancer and glioblastoma, which comprises administration of a pharmaceutical composition of the invention, or of a therapeutically effective amount of an extract of the invention. The subject, as in the definition of the extract of the invention or the pharmaceutical composition of the invention for therapeutic use, can be any mammal, preferably a human being.

Another aspect of the invention refers to the use of the extract of the present invention as an active ingredient to be incorporated into food supplements. "Food supplement" is defined as a product that is added to a diet and that contains substances of natural origin with a nutritional or physiological function that are called functional ingredients. It is generally taken orally and may come in different forms: pills, tablets, capsules, powder sachets, ampoules of liquid, and dropper bottles.

Also considered an aspect of the invention is a food complement or supplement that comprises the extract of the invention as an active or bioactive ingredient. This supplement may also be called a nutraceutical, which is understood as a dietary supplement or complement, presented in a non-food matrix (pills, capsules, powder, etc.), of a concentrated bioactive natural substance, preferably of plant origin, which has a favorable effect on health greater than that which the food itself could have. In other words, nutraceuticals are the components of the food, or parts of it, that provide a proven and added benefit for health, capable of providing medical improvements, in the prevention and treatment of diseases such as colorectal cancer, pancreatic cancer and glioblastoma.

### Description of the drawings

Fig. 1.- Shows a diagram of the methodology for obtaining an ethanolic extract from seed meal.
Fig. 2.- Shows a representative image of the macroscopic difference between ethanolic extracts of the meal of mature seeds of *Solanum melongena* sample S0506 without defatting (a), and of the mature seeds of *Solanum melongena* sample S0506 previously defatted (b) based on the different presence of lipid compounds.
Fig. 3.- Shows a graph of the chromatography of the ethanolic extracts from the seed meal a) without defatting and b) previously defatting *Solanum melongena* sample S0506.
Fig. 4.- Shows a graph of the chromatography of the ethanolic extracts from defatted meal of the *Solanum melongena* samples: a) S0504, b) S0505, c) S0503.
Fig. 5.- Shows the development of Western Blot membranes for Caspase 3, 8 and 9 expression in cells of the T84 colon tumor line treated with an IC₅₀ of the ethanolic extract from defatted mature seed meal of *Solanum melongena* S0506, as well as in control cells (cells of the T84 colon tumor line without treatment).
Fig. 6.- Shows a representation of the expression level of Caspases 3, 8 and 9 in T84 colon tumor cells treated with an IC₅₀ of the ethanolic extract from defatted mature seed meal of *Solanum melongena* S0506.
Fig. 7.- Shows images obtained by fluorescence microscopy of the effect of the ethanolic extract of defatted meal of *Solanum melongena* S0506 on the polymerization/depolymerization of microtubules in T84 cells, using immunofluorescence with the antibody for α-tubulin.
Fig. 8.- Shows images obtained by fluorescence microscopy of T84 cells treated with ethanolic extract from defatted meal of *Solanum melongena* S0506, in which the formation of autophagic vesicles is observed using the LysoTracker^{(R)} assay (highly selective probe for acid organelles consisting of a fluorophore linked to a weak base).
Fig. 9.- Shows images obtained by fluorescence microscopy of the formation of blood vessels by HUVEC cells in the presence of various conditioned media.
Fig. 10.- Shows the graphical representation of the segments, segment size, nodes formed, junctions and meshes (months) formed during angiogenesis by HUVEC cells after exposing them to different conditioned media.
Fig. 11.- Shows the representation of the relative expression values of seven cell markers of tumor stem cells (CD133, CD24, CD44, SOC2, OCT4 and NANOG) quantified by qPCR.

### Detailed description of the invention

The present invention is based on the methodology for obtaining and the in-depth analysis of ethanolic extracts from a plant species of the genus *Solanum* (Solanaceae), more specifically, obtained from the meal of mature seeds of *Solanum melongena* (eggplant).

At present, extracts obtained from different parts (sepals, roots, fruits, fruit skin, etc.) of *Solanum melongena* can be found in the existing literature. Such extracts are generally obtained with highly toxic organic solvents and/or extraction processes of great complexity and duration that can increase the toxicity of the wide variety of components (flavonoids, coumarins and terpenoids) produced. This is the case, for example, in the study by Kintia and Shvets in 1985, commented on in the previous "Background of the invention" section, in which an extract from the seeds of said plant was obtained, but in which methanol was used under reflux, a compound known to have a toxicity greater than that of ethanol at lower concentrations, and to potentially cause dizziness, nausea, vomiting, damage to the nervous system and even death. In this study, several new saponins were isolated and characterized, but they were not reported to have any activity against colon, pancreatic, or glioblastoma cancer. In fact, there are currently no studies of bioactive compounds with antitumor activity against colon cancer obtained from *Solanum melongena* seeds.

Moreover, studies of extracts from other parts of the plant (sepals, roots, etc.) obtained with refluxing ethanol have focused on the isolation of certain compounds present in the extracts and, in the case of having carried out antiproliferative tests, on testing the possible cytotoxicity of said compounds, individually, against certain cancer lines, among which, in general, colon, pancreatic or glioblastoma cancer lines were not included.

In response to the possible drawbacks presented by the extracts from different parts of the plant described in the prior art, the present inventors have developed an extract that exhibits favorable bioactivity and can be obtained from mature seeds of the plant, using simple extraction processes of short duration with non-toxic solvents, while allowing adequate extraction yields. This new extract also presents a more specific composition of active and low-toxic substances that are the *Solanum melongena* polyphenols.

The present invention is based on:
1) The development of an ethanolic extract from *Solanum melongena* mature seed meal which, once analyzed, has a high polyphenol content. The extract was obtained from the meal of the mature seeds, without defatting, and from the meal of the mature seeds, previously defatted, after a cold ethanolic extraction process (at a temperature between 0 ºC and 8 ºC, preferably at 4 ºC) of short duration (preferably, with a maximum of 1 - 2 total hours of contact time of the ethanol with the meal and the possible pellet resulting from a first extraction process), under a nitrogen atmosphere and in an acid medium, which is largely composed of polyphenolic compounds dissolved in a low toxicity solvent.
2) The potent antitumor activity of *Solanum melongena* seed extracts appears when tested in human and murine colon cancer cell cultures (T84 of metastatic human colorectal cancer, HCT15 of colorectal cancer with multidrug resistance (MDR) mechanisms, and MC38 of murine colorectal cancer developed in C57BL6 mice) using the HepG2 line of human hepatocytes as a control, also allows the determination of their therapeutic range. Similarly, the mechanisms of action by which the extracts act on tumor cells were studied to elucidate the molecular pathways that activate cell death.

The results obtained from the research disclosed in this application show that:
1) The methodology used to obtain the ethanolic extract (hydroalcoholic extraction) from the meal from mature, non-defatted and defatted, seeds of *Solanum melongena,* results in a product with a high polyphenol content.
2) The defatting methodological processing, which corresponds to preferred embodiments of the present invention, represents an alternative with some additional advantages for the further development of the ethanolic extract of the present invention. The defatting process will allow, on the one hand, the concentration, in the defatted meal, of polyphenols with a high bioactive capacity that ensures a high extraction yield, and, on the other hand, the elimination of diterpenoid compounds that can be toxic. Even so, as can be seen in Example 2 below, in the tests carried out with different cell lines derived from colon cancer, both the extracts from the meals from non-defatted mature seeds and from defatted mature seeds have great antiproliferative activity, presenting very low IC₅₀ in both cases, which allows considering the use of both extracts for the preparation of pharmaceutical compositions. This idea is reinforced by the fact that both extracts also show antiproliferative capacity against other types of cancer, such as glioblastoma multiforme or pancreatic adenocarcinoma, therefore both extracts, or pharmaceutical compositions prepared therefrom, could also be used for the treatment of these other two types of cancer.
3) The ethanolic extract from the meal (both non-defatted and defatted) of the mature seeds of different varieties of *Solanum melongena* has a high antitumor activity, specifically on cells derived from colon cancer, both resistant and non-resistant to chemotherapy, as well as on murine cells, inducing a potent antiproliferative effect, which was not observed in human hepatocyte cells, thus showing its wide therapeutic range.
4) The IC₅₀ values (concentration of an agent that inhibits a cellular biological process by 50%, a process that, in the tests of the present application, is cell proliferation) observed in human adenocarcinoma lines when tested against ethanolic extracts obtained from *Solanum melongena* mature seeds are very low and, therefore, suitable to be tested for potential use against colorectal cancer, pancreatic cancer and/or glioblastoma.
5) The antiproliferative effect of the ethanolic extract from *Solanum melongena* seed meal, both defatted and non-defatted, also has high antitumor activity against glioblastoma multiforme cell lines, even cell lines resistant to chemotherapy; bearing in mind that this is one of the most aggressive cancers, which presents greater resistance to the cytotoxic drugs commonly used in clinic practice and which currently has the smallest therapeutic arsenal, this property of the ethanolic extract can be considered of special clinical interest. Antiproliferative effects are also observed against pancreatic cancer cell lines, which makes it possible to consider that the ethanolic extracts obtained from these seeds can be used for this therapeutic application.
6) The antitumor activity of the functional ethanolic extract from the defatted seeds of *Solanum melongena,* specifically that obtained from sample S0506, involves the activation of the caspase pathway, both the intrinsic and extrinsic pathways, producing cell death by apoptosis. Furthermore, this extract induces cell death from a disorder in the polymerization/depolymerization of cell microtubules.
7) The antitumor activity presented by the same ethanolic extract of point 6), obtained from defatted seeds of *Solanum melongena,* also involves, although to a lesser extent, the activation of autophagic pathways to induce cell death.
8) According to the tests carried out with the extract obtained from sample S0506, the ethanolic extract from defatted mature seeds of *Solanum melongena* prevents a correct communication of the tumor cells with the endothelial cells, thus decreasing the angiogenesis process, and, thus, the formation of blood vessels.
9) According to the tests carried out with the extract obtained from sample S0506, the ethanolic extract from defatted mature seeds of *Solanum melongena* selectively decreases the population of cancer stem cells (CSCs) present in colon cancer, cells that are responsible for the tumor recurrences and the phenomena of resistance to treatments.
10) The chromatographic analysis, coupled to mass spectrometry, carried out on the extracts obtained from each of the samples (Example 1), shows that there are compounds that appear in most of the extracts, among which some compounds previously isolated from various plants for which biological activity had already been described, such as salannin, capsianoside II, myricomplanoside, aryllatose B, and kaempferol 3-sophorotrioside. Thus, as previously mentioned, in individual trials, salannin showed melanogenesis inhibitory activity and antiproliferative activity against osteosarcoma and neuroblastoma lines. Myricomplanoside was associated with antioxidant, anti-inflammatory, and antiproliferative activity, while capsianosides are characterized by interfering with cytoskeletal function, and kaempferol 3-sophorotrioside is associated with antioxidant and hepatoprotective activity.

Thus, the ethanolic extraction methodology provided by the present invention allows obtaining a non-toxic extract for use in biomedicine, particularly as an antitumor agent. Said methodology represents an enormous advantage for the purpose of its application in patients, since it is based on the use of ethanol, a solvent that is used regularly and at low concentrations for the administration of active principles.

### Examples

Given the scientific interest that underlies the *Solanum melongena* species, ethanolic extracts from mature seeds of different varieties of this species, donated by the plant breeding company Agrointec Solutions SL, belonging to the Cellbitec Business Group (www.cellbitec.com) were studied as described in the trials presented below.

Seeds of four varieties of different types of the *Solanum melongena* species were used, covering the widest spectrum of characterization of this species, following the recommendations of the International Union for the Protection of New Varieties of Plants (UPOV, www.upov.int) defined in document TG/117/4 (https://www.upov.int/edocs/tqdocs/es/tq117.pdf), the samples being those listed below:
1. Variety S0503, Eggplant (*Solanum melongena*): This variety was improved and developed by the donating company, its typological characterization being a semi-long fruit, of uniform size, few seeds, small calyx and without spikes, with an average weight of 300 to 400 gr., bright skin color with purple on white stripes; plant with balanced medium-high vigor, with good production in cold conditions and resistance to the Tobacco Mosaic Virus or TMV. The seed sample analyzed belongs to production batch No. L1 -S0503.
2. Variety S0504, Eggplant (*Solanum melongena*): This variety was improved and developed by the donating company, its typological characterization being a semi-long fruit, of great uniformity, which can present spikes in the calyx under cold conditions, with an average weight of 350 to 500 gr., skin color black, maintaining quality until the end of winter; with a medium vigor plant, it is open and with little emission of secondary flowers, it sets easily throughout its entire cycle and adapts well to long cycles. Suitable for cultivation in greenhouses, tunnels and outdoors. The seed sample analyzed belongs to production batch No. L1 70504.
3. Variety S0505, Eggplant (*Solanum melongena*): This variety was improved and developed by the donating company, its typological characterization being a cylindrical fruit without spikes, with an average weight of 40 to 60 gr., growth in the form of a bouquet with between 5 and 7 fruits, white and shiny skin color; high vigor plant, with good tolerance to stress situations of high and low temperatures, good tolerance to diseases and insects. The seed sample analyzed belongs to production batch No. L170505
4. Variety S0506, Eggplant (*Solanum melongena*): This variety was improved and developed by the donating company, its typological characterization being a semi-long fruit, of uniform size, with few seeds, a small calyx and spikes, with an average weight of 300 at 450 gr., skin color shiny black; very balanced medium vigor plant, with easy access for fruit harvest, recommended for greenhouse cultivation due to its parthenocarpic nature. The seed sample analyzed belongs to production batch No. L1-S0506.

### Example 1. Methodology for obtaining the ethanolic extract and analysis

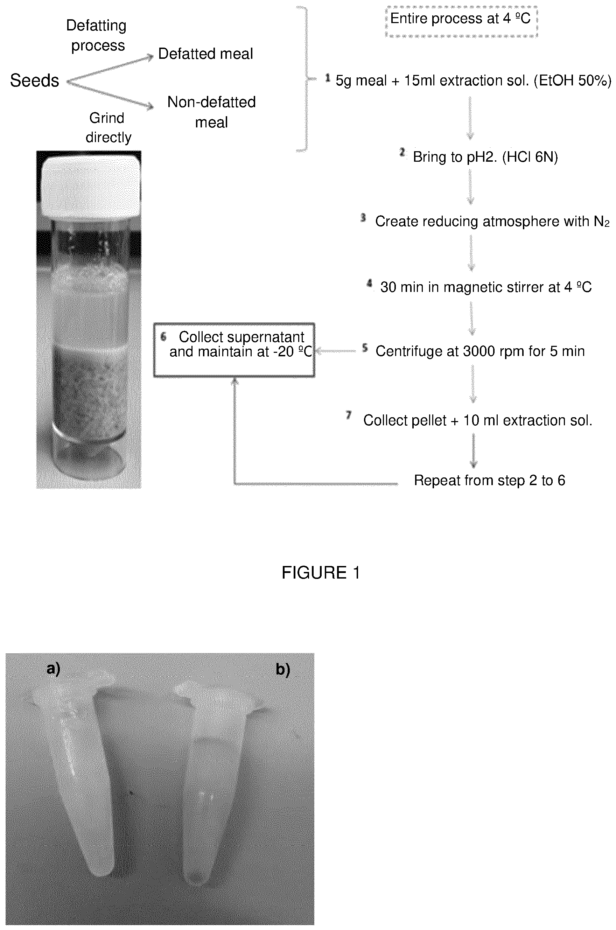

The method for obtaining the ethanolic extract that was developed from the meal of the mature seeds, both non-defatted and defatted, of *Solanum melongena,* is schematized in Fig. 1 and was as follows:
1. Depending on the case:
   a) In the case of non-defatted meal: grind the mature seeds to obtain meal, which is kept at -20 ºC.
   b) In the case of defatted meal: the oily part of the mature seed is separated from the solid part or pellet, for which a KOMET series seed oil extraction press is used which is characterized by its special cold pressing process in which, instead of individual compression screws, screw conveyors are used to squeeze out the oil. In this machine, the oilseeds are gently pressed without exceeding 50 degrees Celsius. With an average working speed of 2-3 kg of seeds per hour and a conversion yield from oil to seed between 15-25%. As a result, a compact and defatted dry cake is obtained. This cake is then ground at 28,000 rpm to obtain a defatted meal with a particle size of between 100 and 150 microns, which is subsequently stored at -20 ºC.
2. Weigh 5 g. of 1.a) or 1.b) in a beaker and immediately place the beaker on ice to work at a temperature of 4 ºC.
3. Add 15 ml of extraction solution (50% ethanol (EtOH) = 50 ml EtOH + 50 ml bidistilled water + 0.2 ml hydrochloric acid (HCl 12N).
4. Stir in a magnetic stirrer at 300 rpm.
5. Bring to pH 2 (by adding 6N HCl).
6. Add gaseous nitrogen so that there is no oxidizing environment and the polyphenols are properly preserved.
7. Leave stirring for 30 min. at 4 ºC.
8. Centrifuge at 3000 rpm for 5 min. at 4 ºC.
9. Collect the supernatant and store it at -20 ºC.
10. The precipitate is resuspended in 10 ml of extraction solution and a second extraction is carried out following the steps indicated above.
11. Finally, after carrying out the pertinent extractions, the supernatants of each extraction are pooled and stored at -20 ºC until use.
12. The pellet originated in the last centrifuge is discarded.

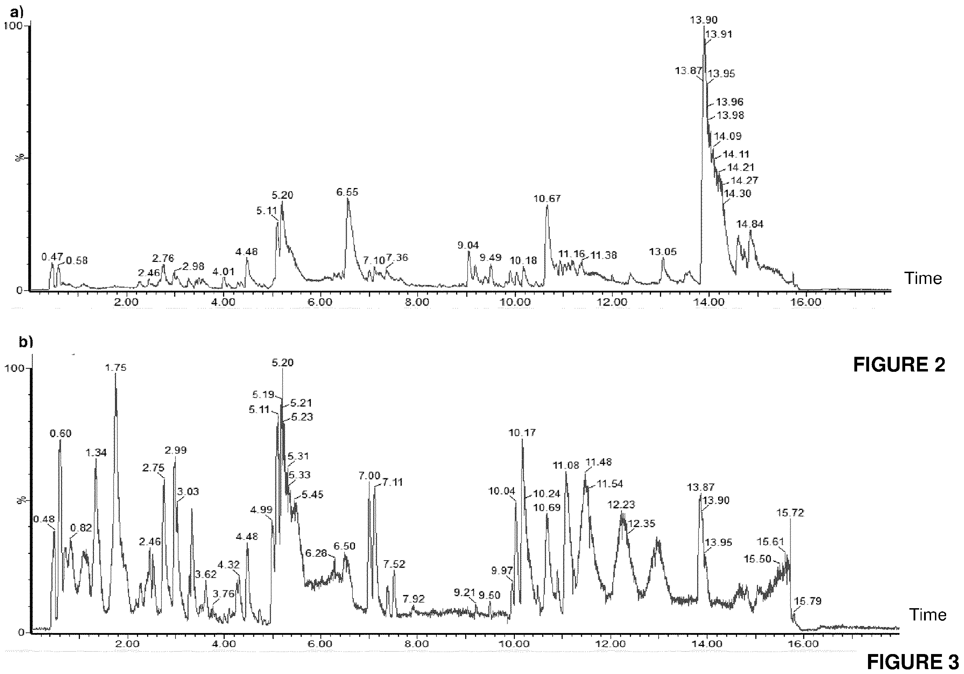

Following the extraction protocol described, ethanolic extracts were obtained from the defatted and non-defatted mature seed meal of *Solanum melongena.* The objective of this double process was to compare the activity of the extracts, removing the high percentage of lipid compounds from the mature seeds without defatting (see Fig. 2), where the macroscopic difference between both ethanolic extracts can be appreciated based on the different presence of lipid compounds) and to compare their functional activity against the extracts obtained from previously defatted mature seed meal.

To determine the yield, the ethanolic extract was divided into 1 mL aliquots for ethanol evaporation and subsequent lyophilization of the remaining water in the extract. In order to remove the ethanol from the ethanolic extracts, it was evaporated under vacuum using a Savant DNA 120 evaporation system (Thermo Scientific) for 60 minutes. After evaporation of the ethanol, the aliquots were frozen in liquid nitrogen with the remaining extract and lyophilized using a TELSTAR Cryodos-50 lyophilizer where they were kept for 24 hours. After lyophilization, the dry weight of the extract was calculated based on the difference with the container that contained each aliquot and said dry weight was referred to a volume of 1 mL of initial extract, subsequently to the total volume of extract obtained, and finally to the grams of starting seed meal for the preparation of the extract.

Total polyphenols were determined by means of the Dewanto et al. technique (2002), as described by Kapravelou et al. (2015), which uses a standard line of gallic acid with concentrations between 0 and 500 µg/mL. Furthermore, the reducing capacity of Fe3+ to Fe2+ by the different extracts was determined spectrophotometrically by means of Duh et al. (1999) technique, as described by Kapravelou et al. (2015), which uses a standard line of gallic acid with concentrations also between 0 and 500 µg/mL.

Table 1 shows the yield and total polyphenols of the extract from the non-defatted and defatted mature seed meal of the *Solanum melongena* S0503, S0504, S0505 and S0506 varieties. As can be seen in the aforementioned table, the yields obtained in the analyzed cases, for all the varieties of *Solanum melongena,* showed a great variability, with those of the extracts from defatted mature seeds being significantly higher than those of the extracts from the non-defatted mature seeds.

**Table 1. Yield values (mg/g meal) and total polyphenols (µg gallic acid equivalent/mg extract) of the ethanolic extracts from different varieties of non-defatted and defatted mature seeds of Solanum melongena (S0503, S0504, S0505 and S0506)**

| Varieties of *Solanum melongena* | Yield (mg/g meal) | Total polyphenols (µg gallic acid equivalent/mg extract) |
|---|---|---|
| NON-DEFATTED S0503 | 30.90 | 19.95 |
| DEFATTED S0503 | 86.79 | 27.59 |
| NON-DEFATTED S0504 | 35.48 | 15.23 |
| DEFATTED S0504 | 45.60 | 15.71 |
| NON-DEFATTED S0505 | 39.68 | 14.44 |
| DEFATTED S0505 | 64.80 | 12.06 |
| NON-DEFATTED S0506 | 42.60 | 11.16 |
| DEFATTED S0506 | 71.12 | 18.41 |

Furthermore, the antioxidant capacity was analyzed including biochemical studies of total polyphenols and reducing capacity. Regarding total polyphenols, the ethanolic extracts from defatted and non-defatted meal, for all the varieties tested, present very homogeneous values, being, in general, and with the exception of sample S0505, higher in the ethanolic extracts from defatted mature seeds (with values ranging between 12.06 and 27.59 µg gallic acid equivalent/mg extract for the ethanolic extract from common eggplant from defatted mature seeds) than in the ethanolic extracts from non-defatted meal (11.16 µg gallic acid equivalent/mg extract in S0506). These results made it possible to corroborate that the process of elimination of lipidic compounds, in addition to increasing the performance of the functional extracts, does not alter or eliminate the bioactive compounds of interest; additionally, they confirmed that the above mentioned method made it possible to purify the functional extract.

The results obtained from sample S0506 deserve to be highlighted (which is done throughout the present specification), as said sample has one of the highest yields (42.6 mg/g meal in the ethanolic extract from non-defatted mature seeds and 71.12 mg/g meal in the ethanolic extract from defatted seeds). The value of total polyphenols determined in the ethanolic extract from defatted mature seed meal (18.41 µg gallic acid equivalent/mg extract) is one of the highest among the extracts tested. In addition, as will be seen later in Example 2, it is one of those with the greatest antitumor activity (see Table 10). For all these reasons, as will be mentioned later, it was selected to be the subject of specific studies carried out to elucidate the molecular mechanisms of cellular action.

The reducing capacity tests for the biochemical determination of the antioxidant capacity were carried out with the extracts selected from sample S0506, obtaining values of 5.3 µg gallic acid equivalents/mg extract for the extract from non-defatted mature seed meal, and 6.31 µg gallic acid equivalents/mg extract for the ethanolic extract from defatted meal (Table 2). Therefore, the antioxidant capacity of the extracts prepared with defatted mature seed meal was higher based on the fact that the polyphenols are obtained in greater quantities.

**Table 2. Values of reducing capacity (µg gallic acid equivalent/mg extract) of the ethanolic extract from non-defatted and previously defatted mature seeds of sample S0506**

| Variety of *Solanum melongena* | Reducing capacity (µg gallic acid equivalent/mg extract) |
|---|---|
| Non-defatted S0506 | 5.33 |
| Defatted S0506 | 6.31 |

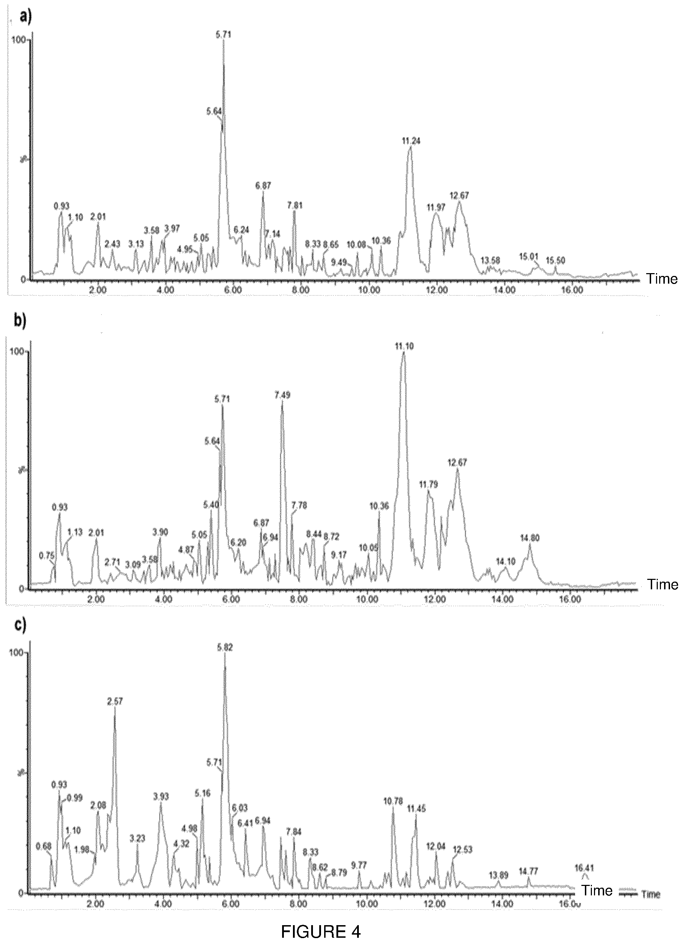

The chromatographic studies carried out made it possible to determine the compounds present in the ethanolic extract from the mature seed meals of *Solanum melongena.* These studies used Waters ACQUITY H-Class Ultra Performance Liquid Chromatography (UPLC) together with a Waters SYNAP G2 Q-TOF Mass Spectrometer. Figs. 3 and 4 show the graphs of the chromatography of the ethanolic extracts obtained, both from the defatted and non-defatted mature seed meal of S0506 (Fig. 3), and the defatted mature seed meal of the other samples of *Solanum melongena* (S0503, S0504, S0505) (Fig. 4).

Tables 3, 4, 5, 6 and 7 below indicate the main compounds identified in each of the extracts of the *Solanum melongena* varieties and the chromatographic data of each of them (for compounds with more than one chromatographic peak, only the data of one of the peaks is indicated). Said compounds, and their number in the CAS (Chemical Abstract Service) registry are: triglycidyl trimellitate (CAS No: 7237-83-4), myricomplanoside (CAS No.: 123442-26-2), Kaempferol 3-sophorotrioside (CAS No.: 80714-53-0), Arylatose B (CAS No.: 137941-45-8), Quercetin 3-rhamninoside (CAS No.: 522-12-3), Swertiajaponin 3'-O-gentiobioside (CAS No.: 76166-51-3), Quercetin 3,7-diglucoside (CAS No.: 6892-74-6), Quercitrin (CAS No.: 522-12-3), Ramontoside (CAS No.: 133882-75-4), Macrostemonoside J (CAS No.: 159935-09-8), Salannin (CAS No.: 992-20-1), Mudanpioside J (CAS No.: 262350-52-7), Cudranian 1, Capsianoside III (CAS No.: 121961-81-7), Protodioscin (CAS No.: 55056-80-9), Capsianoside II (CAS No.: 121924-04-7), Macrantoside I (CAS No.: 90850-94- 5), Dioscin (CAS No.: 19057-60-4), Asparanin D (CAS No.: 83931-89-9), Kalambroside A (CAS No.: 160472-99-1), Citrusin B (CAS No. : 105279-10-5), Chlorogenic acid (CAS No.: 327-97-9), Quercetin-3,4'-O-di-β-glucopyranoside (CAS No.: 29125-80-2), Myricitrin (CAS No.: 17912-87-7), Macrostemonoside J (CAS No: 159935-09-8), Blumeoside C (CAS No: 94657-28-8), Pikuroside (CAS No: 231280-24-3), Kaempferol 3-(2G-xylosylrutinoside)-7-glucoside (CAS No: 131559-51-8), Gypenoside LVI (CAS No: 109145-67-7), Vismione D (CAS No: 87605-72-9), Volubiloside B (Cas No: 485807-79-2), Nonadecanoic Acid (CAS No: 646-30-0).

**Table 3. Bioactive compounds of the ethanolic extract from non-defatted mature seed meal of Solanum melongena, variety S0506**

| Name | MF | PPM | RT | [M-H]- | Conf% |
|---|---|---|---|---|---|
| Triglycidyl trimellitate | C18H18O9 | -6.1 | 0.58 | 377.085 | 87.78 |
| Myricomplanoside | C22H22O13 | 7.9 | 2.46 | 493.1021 | 99.74 |
| Kaempferol 3-sophorotrioside | C33H40O21 | 1.6 | 2.46 | 771.1996 | 100 |
| Quercetin 3-rhamninoside | C33H40O20 | 2.9 | 2.76 | 755.2057 | 99.96 |
| Swertiajaponin 3'-O-genthiobioside | C34H42O21 | 2.7 | 2.76 | 785.2161 | 97.22 |
| Arillatose B | C22H30O14 | -8 | 2.98 | 517.1553 | 98.87 |
| Quercetin 3,7-diglycoside | C27H30O17 | 4 | 2.98 | 625.143 | 99.55 |
| Quercitrin | C21H20O11 | 6.5 | 4.01 | 447.0956 | 97.68 |
| Ramontoside | C34H38O16 | 1.4 | 4.01 | 701.2092 | 99.84 |
| Macrostemonoside J | C45H76O20 | -1.2 | 4.48 | 935.4841 | 90.22 |
| Capsianoside II | C50H84O25 | -7.9 | 5.2 | 1083.5137 | 85.84 |
| Salannin | C34H44O9 | -1.2 | 11.6 | 595.29 | 98.45 |

| | | | | | |
|---|---|---|---|---|---|
| RT: retention time; MF: molecular formula; PPM: error; MS: mass; Conf%: confidence percentage. | | | | | |

**Table 4. Bioactive compounds of the ethanolic extract from defatted mature seed meal of Solanum melongena, variety S0506.**

| Name | MF | PPM | RT | [M-H]- | Conf% |
|---|---|---|---|---|---|
| Mudanpioside J | C31H34O14 | -3 | 1.34 | 359.1851 | 99.71 |
| Kaempferol 3-sophorotrioside | C33H40O21 | 3.9 | 2.46 | 771.2014 | 99.58 |
| Myricomplanoside | C22H22O13 | 7.5 | 2.46 | 493.1019 | 91.22 |
| Arillatose B | C22H30O14 | 0.8 | 2.75 | 517.1561 | 99.95 |
| Cudranian 1 | C28H26O12 | -3.3 | 2.75 | 553.1328 | 99.36 |
| Quercetin 3,7-diglycoside | C27H30O17 | 1.9 | 2.99 | 625.1417 | 99.95 |
| Ramontoside | C34H38O16 | 5.1 | 3.62 | 701.2118 | 99.56 |
| Capsianoside III | C50H84Q26 | -7.6 | 5.2 | 1099.5089 | 99.98 |
| Protodioscin | C51H84O22 | 3.2 | 5.2 | 1047.514 | 78.86 |
| Capsianoside II | C50H84Q25 | -8.1 | 6.5 | 1083.5135 | 99.54 |
| Macrantoside I | C45H74O19 | -2 | 6.5 | 917.4728 | 99.86 |
| Asparanine D | C50H82O21 | -1.1 | 7.11 | 1017.5259 | 99.9 |
| Dioscine | C45H72016 | 0.9 | 9.5 | 867.475 | 99.07 |
| Protodioscin | C51H84O22 | 0.4 | 10.17 | 1047.538 | 97.27 |
| Salannin | C34H44O9 | -3.2 | 11.48 | 595.2888 | 95.17 |

| | | | | | |
|---|---|---|---|---|---|
| RT: retention time; MF: molecular formula; PPM: error; MS: mass; Conf%: confidence percentage. | | | | | |

**Table 5. Bioactive compounds of the ethanolic extract from defatted mature seed meal of Solanum melongena, variety "S0503"**

| Name | MF | PPM | RT | [M-H]- | Conf% |
|---|---|---|---|---|---|
| Triglycidyl trimellitate | C18H18O9 | 8.8 | 0.99 | 377.0906 | 94.2 |
| Mudanpioside J | C31H34O14 | -4.6 | 1.1 | 629.1841 | 98.48 |
| Mudanpioside J | C31H34O14 | 0.8 | 2.57 | 629.1875 | 99.98 |
| Myricomplanoside | C22H22O13 | 9.3 | 3.23 | 493.1028 | 99.94 |
| Capsianoside II | C50H84O25 | -1.7 | 5.82 | 1083.5205 | 95.55 |
| Calambroside A | C32H36O18 | 6.1 | 3.93 | 707.1866 | 99.82 |
| Citrus B | C27H36O13 | 0.8 | 4.32 | 567.2105 | 93.89 |
| Ramontoside | C34H38O16 | 1 | 5.16 | 701.2089 | 99.67 |
| Capsianoside II | C50H84O25 | -0.2 | 5.75 | 1083.5221 | 99.69 |
| Salannin | C34H44O9 | -3.4 | 10.78 | 595.2880 | 94.29 |

| | | | | | |
|---|---|---|---|---|---|
| RT: retention time; MF: molecular formula; PPM: error; MS: mass; Conf%: confidence percentage. | | | | | |

**Table 6. Bioactive compounds of the ethanolic extract from defatted mature seed meal of Solanum melongena, variety "S0504".**

| Name | MF | PPM | RT | [M-H]- | Conf% |
|---|---|---|---|---|---|
| Blumeoside C | C24H28O14 | 8.3 | 0.93 | 539.1446 | 99.8 |
| Pikuroside | C23H30O14 | 0.9 | 3.13 | 529.1562 | 99.81 |
| Myricomplanoside | C22H22O13 | 4.7 | 3.13 | 493.1005 | 90.01 |
| Kaempferol 3-(2G-xylosylrutinoside)-7-glucoside | C38H48O24 | 4.5 | 3.97 | 887.2497 | 98.06 |
| Capsianoside II | C50H84O25 | -1.1 | 5.71 | 1083.5211 | 98.03 |
| Gynostemma P.E. | C53H90O23 | 1.3 | 6.24 | 1093.5809 | 94.47 |
| Salannin | C34H44O9 | -5.9 | 11.24 | 595.2872 | 96.35 |

| | | | | | |
|---|---|---|---|---|---|
| RT: retention time; MF: molecular formula; PPM: error; MS: mass; Conf%: confidence percentage. | | | | | |

**Table 7. Bioactive compounds of the ethanolic extract from defatted mature seed meal of Solanum melongena, variety "S0505".**

| Name | MF | PPM | RT | [M-H]- | Conf % |
|---|---|---|---|---|---|
| Myricomplanoside | C22H22O13 | 5.9 | 3.09 | 493.1011 | 97.86 |
| Arillatose B | C22H30O14 | -1.2 | 3.58 | 517.1551 | 98.84 |
| Kaempferol 3-(2G-xylosylrutinoside)-7-glucoside | C38H48O24 | 0.9 | 3.9 | 887.2465 | 97.8 |
| Capsianoside II | C50H84O25 | -2.2 | 5.71 | 1093.5199 | 97.36 |
| Vismione D | C25H30O5 | -5.6 | 6.2 | 409.1992 | 94.56 |
| Volubiloside B | C47H78O20 | 6.3 | 8.44 | 961.5069 | 89.45 |
| Nonadecanoic acid | C36H68O10 | -1.5 | 8.72 | 659.4724 | 99.39 |
| Salannin | C34H44O9 | -4.9 | 11.1 | 595.2878 | 95.61 |

| | | | | | |
|---|---|---|---|---|---|
| RT: retention time; MF: molecular formula; PPM: error; MS: mass; Conf%: confidence percentage. | | | | | |

Among the compounds present in the vast majority of the extracts, from both non-defatted and defatted mature seed meals, Salannin (C34H44O9), Kaempferol 3-sophorotrioside (C33H40O21), Myricomplanoside (C22H22O13), Arillatose B (C22H30O14) and Capsianoside II (C50H84O25) stand out (Figs. 3,4,5,6,7). The biological activity of these compounds was studied in isolation.

As it was observed, the ethanolic extracts of the varieties, from both non-defatted and defatted mature seed meals of *Solanum melongena,* share, for the most part, several of the bioactive compounds described above. This is logical, since the different samples used genetically belong to the same plant species and the active compounds are part of important metabolic pathways, although it should be noted that, morphologically, the fruits, as indicated in the UPOV TG/117/4 protocol, can be quite varied while all the phenotypic varieties belong to the same species, that is, *Solanum melongena.*

Due to the lack of studies on the antitumor activity of the bioactive compounds present in the mature seeds of the *Solanum melongena* species in colon cancer, and the limited knowledge about the independent activity of the main compounds that integrate it, the research disclosed in the present application was conducted, not only on the antitumor properties of the ethanolic extracts of the invention in colon cancer, but also focused in depth on the performance of these compounds and their combined action.

### Example 2. Determination of the antitumor capacity of the extracts

In order to determine the antitumor capacity of the extracts, the cell lines T84 (human colon adenocarcinoma cell line), HCT15 (human colon adenocarcinoma cell line resistant to chemotherapy) and MC38 (murine colon adenocarcinoma cell line) were cultured. As a control, the HepG2 line was selected (a human hepatocyte cell line whose great differentiation makes it suitable as a model for the study of human hepatocytes in various types of assays, including assays on drug or active compound activity, in which they are often used as controls).

The ethanolic extracts were previously evaporated to avoid the toxicity caused by ethanol on cell lines. In addition, once evaporated, a portion was lyophilized to learn the amount of extract obtained and quantify its concentration (mg/ml), based on which the different concentrations to be tested will be calculated. The cell cultures were exposed to increasing concentrations of the evaporated ethanolic extracts from both the non-defatted and defatted mature seed meals of all types of *Solanum melongena,* which allowed determining the inhibitory concentration 50 (IC₅₀) (concentration of the extract at which the extract inhibits 50% of the cell population) using the Sulforhodamine B technique. The results obtained are shown in Table 8.

**Table 8. Antitumor capacity of the extracts from non-defatted and defatted mature seed meals of different varieties of Solanum melongena (S0503, S0504, S0505 and S0506) in vitro at 72 hours in different lines of colon cancer.**

| | IC₅₀ (µg/ml) in each cell line indicated | | |
|---|---|---|---|
| Varieties | T84 | HCT15 | MC38 |
| NON-DEFATTED S0503 | 25.71 | 29.68 | 27.58 |
| DEFATTED S0503 | 29.96 | 30.71 | 29.99 |
| NON-DEFATTED S0504 | 25.45 | 39.30 | 32.06 |
| DEFATTED S0504 | 30.10 | 38.60 | 43.78 |
| NON-DEFATTED S0505 | 22.10 | 32.16 | 20.92 |
| DEFATTED S0505 | 25.09 | 36.88 | 28.74 |
| NON-DEFATTED S0506 | 23.88 | 37.07 | 48.58 |
| DEFATTED S0506 | 35.07 | 29.63 | 39.3 |

| | | | |
|---|---|---|---|
| T84: human colon adenocarcinoma cell line; HCT15: human colon adenocarcinoma cell line resistant to chemotherapy; MC38: murine colon adenocarcinoma cell line. IC₅₀: concentration that inhibits 50% of the cells. | | | |

As can be seen in the previous table, for the ethanolic extract from non-defatted mature seeds of the S0506 variety, the IC₅₀s were the following: 23.88 µg/ml in T84, 37.07 µg/ml in HCT15 and 48.58 µg /ml in MC38. For the extracts from defatted mature seed meal of S0506, the IC₅₀s were: 35.07 µg/ml in T84, 29.63 µg/ml in HCT15 and 39.3 µg/ml in MC38. It should be noted that the IC₅₀ of the ethanolic extract from defatted meal of S0506 in the HepG2 human hepatocyte line was 99.87 µg/ml, which leaves an acceptable therapeutic range for conducting further *in vivo* research. Furthermore, very similar IC₅₀s were observed in the rest of *Solanum melongena* varieties.

In view of the results, it can be observed that the ethanolic extracts from non-defatted and defatted mature seed meals of *Solanum melongena* have a high antiproliferative activity, with the IC₅₀ being very low and homogeneous among the different samples tested. This fact is noteworthy, since it indicates that the defatting process does not excessively alter the antitumor capacity of the extract obtained and, in some cases (such as the activity on HCT15 cells of the extract obtained from mature seed meal of sample S0506), even increases it. In addition, the difference between the IC₅₀ of the tumor lines (T84, HCT15 and MC38) and the non-tumor line (HepG2) is relevant, being significantly higher in the latter.

Due to the fact that the extracts from the mature seed meals, both non-defatted and defatted, have a similar antitumor activity and that the extracts of the defatted mature seed meal have a higher yield and a higher concentration of polyphenolic compounds, it was decided to use the ethanolic extract from defatted mature seed meal to carry out the rest of the molecular tests that are detailed below. In addition, the ethanolic extract from defatted meal from sample S0506 was selected for these tests, as a representative of the other extracts, due to its lower IC₅₀ value for the HCT15 line and to its high yield.

Lastly, and based on previous results in colon cancer cell lines, the ethanolic extracts, from both the defatted and non-defatted meals of all varieties, were tested on glioblastoma multiforme and pancreatic adenocarcinoma cell lines. For that purpose, cell lines A-172 and LN-229 (human glioblastoma cell line), SF-268 and SK-N-SH (human chemotherapy-resistant glioblastoma cell lines) in addition to Panc-1 (human pancreatic adenocarcinoma cell line) were cultivated. Using the same method described above, the IC₅₀ of the cell lines under study were determined. The results are presented in Table 9.

**Table 9. Results of the use of ethanolic extract from mature seed meal, without defatting and defatted, of different varieties of Solanum melongena, tested in cell lines of glioblastoma multiforme and pancreatic adenocarcinoma.**

| | IC₅₀ (µg/ml) in each cell line indicated | | | | |
|---|---|---|---|---|---|
| Varieties | SF-268 | SK-N-SH | A-172 | LN-229 | Panc-1 |
| NON-DEFATTED S0503 | 35.41 | 36.48 | 37.43 | 34.84 | 50.24 |
| DEFATTED S0503 | 26.19 | 26.47 | 24.6 | 29.71 | 49.39 |
| NON-DEFATTED S0504 | 44.06 | 48.56 | 49.23 | 45.96 | 41.43 |
| DEFATTED S0504 | 35.60 | 36.12 | 32.78 | 35.15 | 65.67 |
| NON-DEFATTED S0505 | 44.99 | 43.63 | 33.59 | 44.22 | 47.08 |
| DEFATTED S0505 | 28.67 | 29.39 | 26.3 | 27.67 | 44.02 |
| NON-DEFATTED S0506 | 23.89 | 25.70 | 24.15 | 21.22 | 45.29 |
| DEFATTED S0506 | 35.36 | 37.75 | 36.11 | 37.96 | 49.02 |

For the ethanolic extract from non-defatted meal of S0506, the IC₅₀ was 23.89 µg/ml in SF-268, 25.70 µg/ml in SK-N-SH, 24.15 µg/ml in A-172 and 21.22 in LN-229, while in the line derived from pancreatic adenocarcinoma Panc-1, the IC₅₀ was 45.29 µg/ml. For the ethanolic extract from defatted meal of the S0506 variety, the IC₅₀s were higher: 35.36 µg/ml in SF-268, 37.75 µg/ml in SK, 36.11 in A-172, 37.96 in LN -229 and 49.02 in Panc-1. In the remaining samples, in contrast, the extracts obtained from defatted meal generally gave lower IC₅₀ values than those from non-defatted meal.

These results allowed us to conclude that the ethanolic extracts of all *Solanum melongena* varieties, both from defatted and non-defatted meal, in addition to presenting a great antitumor capacity against colon cancer, also have high antitumor activity against cell lines of glioblastoma and pancreatic adenocarcinoma.

### Example 3.- Molecular study of proteins related to cell death

To elucidate the mechanisms of action of the extracts of the present invention, the cell death pathway (apoptosis), mediated by caspases, mainly caspase 8 (extrinsic pathway), caspase 9 (intrinsic pathway) and caspase 3, was studied by means of the Western Blot technique, using endogenous β-actin as control.

For this purpose, cells from the colon tumor line (T84) were cultured with an IC₅₀ of the ethanolic extract obtained from the defatted mature seed meal of S0506 and, after 72 hours, the cells were collected to proceed with the protein extraction.

To carry out the Western Blot assay, 40 µg of protein from the cells treated with the ethanolic extract, as well as from the control cells (T84 without treatment) were loaded on an SDS-PAGE electrophoresis polyacrylamide gel in a Mini-Protean II cell (Bio-Rad, Hercules, CA). Once the proteins were separated by electrophoresis, they were transferred to a nitrocellulose membrane to which 20 V were supplied at room temperature for 1 hour. These membranes were treated with blocking solution (PBS-Tween + 5% powdered milk) for 1 hour, then, after 2 washes with PBS-Tween, they were incubated with the primary antibody [rabbit anti-caspase-3 polyclonal IgGs (dilution 1 :500), anti-caspase-8 (1 :1000 dilution) and anti-caspase-9 (1:1000 dilution); Santa Cruz Biotechnology, Santa Cruz, CA]. They were incubated overnight at 4 °C. Once the incubation time had elapsed, two washings were carried out and incubation took place for 1 hour at room temperature with the peroxidase-conjugated secondary antibody. Finally, the proteins were detected by means of ECL (Enhanced Chemiluminescence) (Bonnus, Amersham, Little Chalfont, UK) (Ortiz et al. 2009).

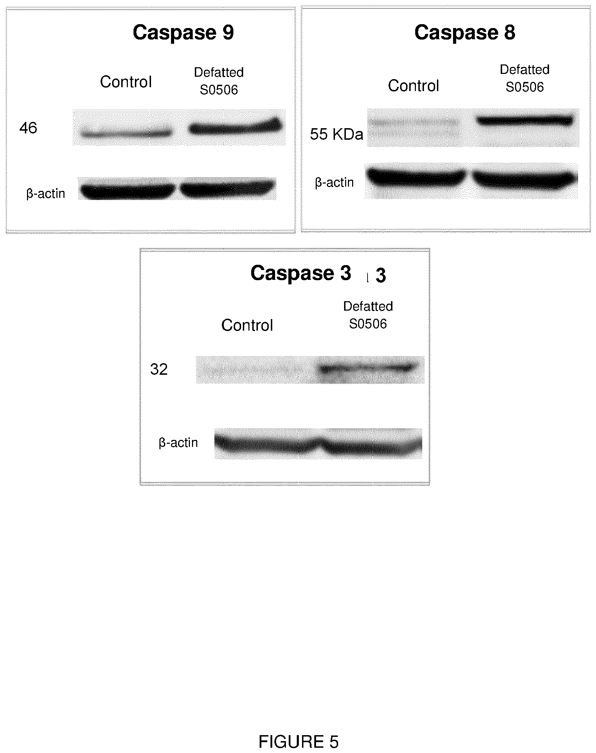

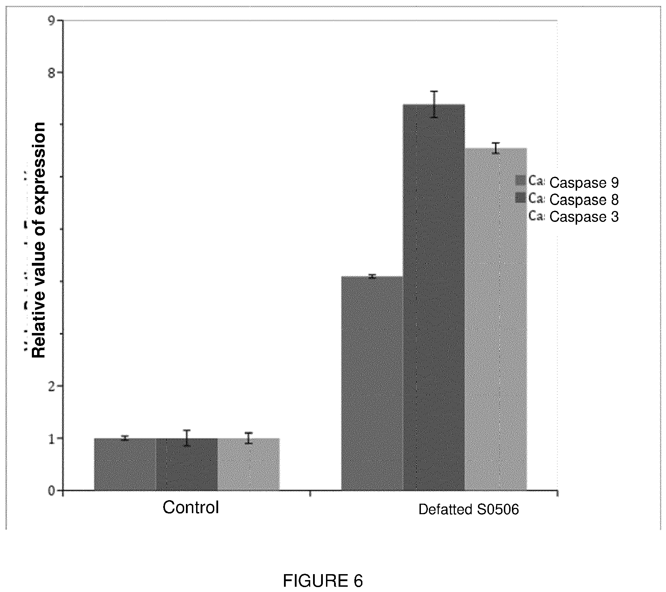

Once the Western Blot was performed (see Fig. 5), the bands obtained in the gels were analyzed using an analytical software called Quantity One by Bio-Rad, which confirmed that the ethanolic extract from the defatted mature seed meal of *Solanum melongena* produces cell apoptosis mediated by the caspase pathway, the caspases 9, 3 and 8 being overexpressed 4, 6.5 and 7.4 times, respectively and in relation to the control (Fig. 6). These data suggest that the ethanolic extract induces cell death by apoptosis, both through intrinsic and extrinsic pathways.

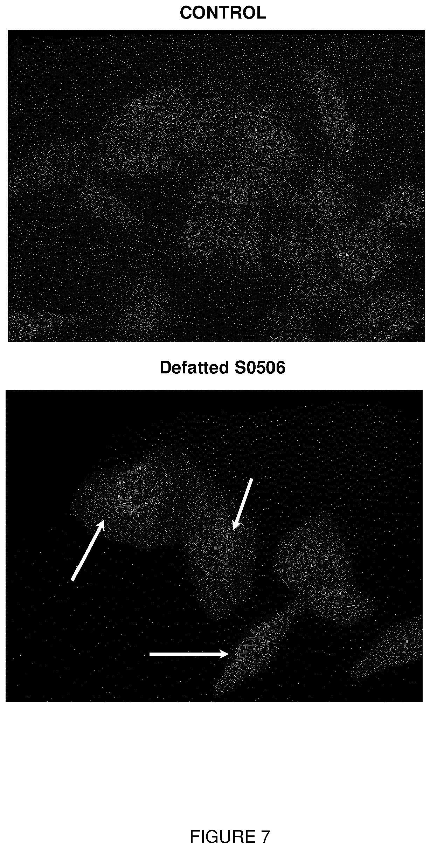

To determine the activation of other possible mechanisms of cell death, studies of possible alterations in the polymerization/depolymerization of cell microtubules were carried out using immunofluorescence. To that end, T84 cell cultures were exposed to the ethanolic extract (IC₅₀) evaporated from the defatted meal of S0506. After 24 hours, the cells were fixed and incubated with an anti-tubulin antibody. As can be seen in Fig. 7, the presence of condensations around the cell nucleus indicates an alteration in the polymerization/depolymerization of the cell microtubules. Some chemotherapeutic agents such as Taxol (from the bark of the yew tree) (Yu et al., 2017) and Vincristine (from the vinca flower) (Wu et al., 2012) act by blocking the polymerization/depolymerization of the tubulin causing the death of tumor cells. These drugs are used in different types of tumors (leukemia, lymphoma, lung cancer, prostate, etc.). However, for colon cancer, there are currently no chemotherapeutics that act at this level. However, the similarity between the extract of the present invention and the action of Vincristine and Taxol is worth noting.

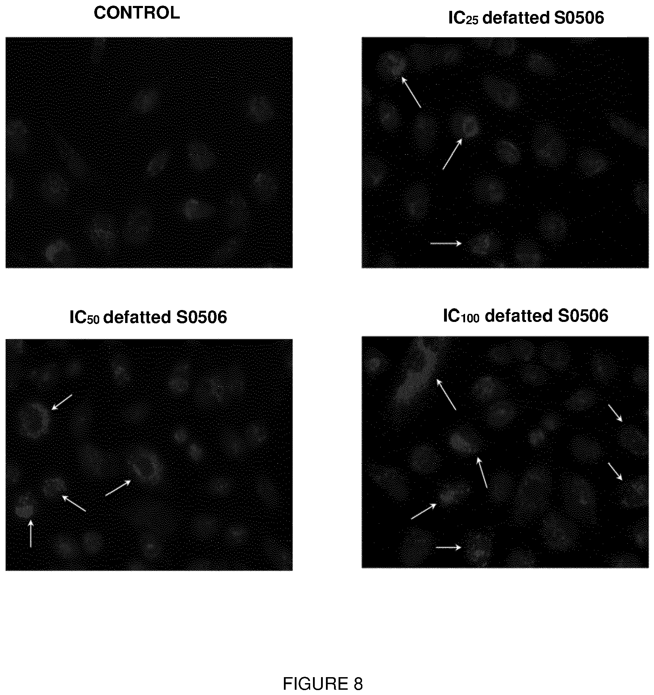

Lastly, despite the fact that most of the cell death induced by the ethanolic extract from the defatted meal of S0506 was produced by apoptotic routes, a certain percentage of it occurred by autophagic routes. Cultures of T-84 cells exposed to the ethanolic extract (24 hours) were stained using a red fluorescent probe (LysoTracker^{™} Red DND-99, ThermoFisher Scientific) and observed under a fluorescence microscope. The appearance of the classic autophagic vesicles in the T84 line indicated the activation of this mechanism (Fig. 8).

### Example 4.- Study to determine the effect on angiogenesis

Angiogenesis or the formation of new blood vessels from endothelial cells is essential for the growth of tumors and for their expansion and the production of metastases. In the present study, the effect of the ethanolic extract on tumor cells in relation to their communication with endothelial cells and, therefore, with the formation of new blood vessels (angiogenesis) was analyzed. T84 cells were incubated with the ethanolic extract from the defatted meal of S0506 at doses of IC₂₅ (20 ug/ml) and IC₅₀ (30 ug/ml) for 24 hours. After washing the wells (PBS) and adding new DMEM (+10% FBS and 1% Ab) free of ethanolic extract, the culture was maintained for 48 hours in order to obtain a conditioned medium. Next, HUVEC cells (umbilical cord endothelial cell line) were seeded in 96-well plates with Matrigel. These cells were contacted with the conditioned media obtained in order to observe the formation of blood vessels.

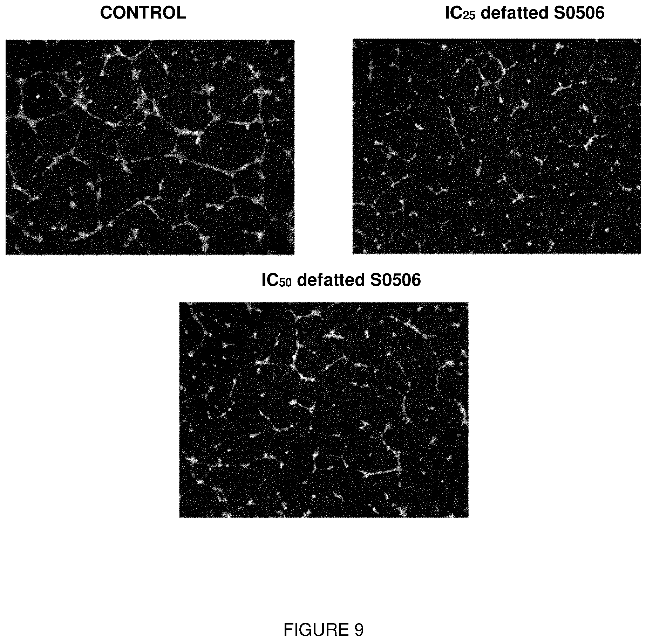

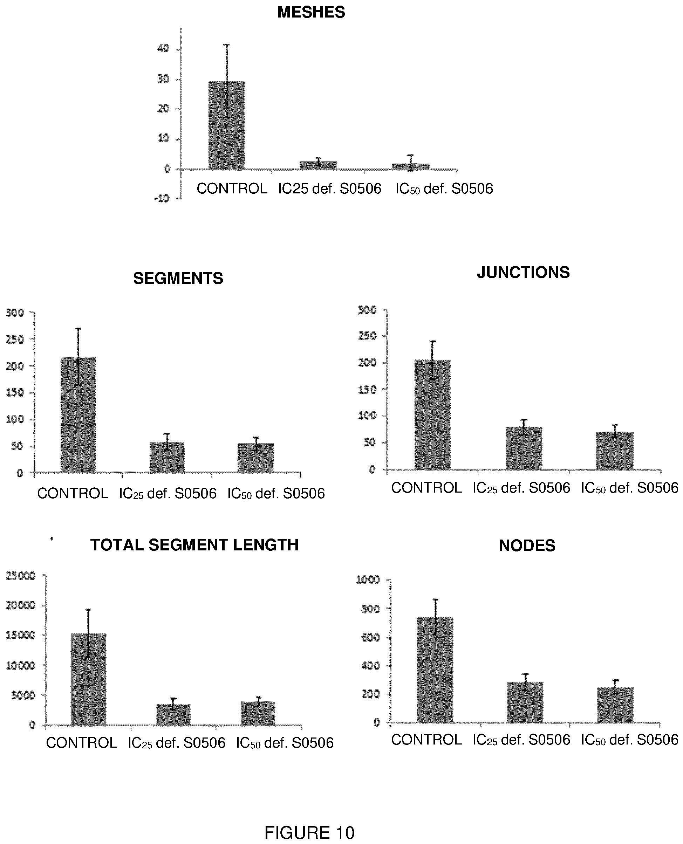

As can be seen in Fig. 9, the conditioned media from T84 cells in contact with the extract inhibited the formation of blood vessels by the HUVEC cells in relation to the control (HUVEC cells without exposure). The quantification of the formation of vessels (number of segments, junctions, nodes formed and length of the segments) demonstrated a significant reduction in relation to the control (Fig. 10). These results suggest that the functional ethanolic extract exerts an effect on tumor cells, modifying the factors with which they interact with epithelial cells and causing a decrease in the angiogenesis process. This result is of enormous importance since it would imply that the ethanolic extract of the invention is capable of inhibiting the formation of new blood vessels that nourish the tumor and, therefore, reduce its growth and metastatic expansion.

### - Example 5.- Studies on the population of cancer stem cells (CSCs) of colon cancer

Tumors are characterized by having subpopulations of tumor cells called cancer stem cells (CSCs), whose fundamental properties are being undifferentiated and pluripotent, but which are especially characterized by having a high tumorigenic capacity and presenting resistance to treatments (chemoresistance). These CSCs are responsible, to a large extent, for clinical recurrences and for the low effectiveness of treatments with therapeutic regimens that are currently used. A large part of the most advanced lines of research in the field of basic and clinical oncology are based on the development of antitumor agents that, preferentially or selectively, affect this cell type, which would guarantee a better patient response to treatment and a greater benefit in patient prognosis. CSCs are a very heterogeneous population that are identified by certain cell markers, such as CD133, CD44, CD24, NANOG, SOX-2 and OCT-4, among others (Munro et al., 2018).

To determine the antitumor capacity of the ethanolic extract of the invention in certain subpopulations of cancer stem cells, T84 cell cultures were exposed to the ethanolic extract (IC₅₀) of the defatted meal of S0506 for 72 hours. From the total RNA extracted from the cultures, a Real Time PCR was carried out to determine the expression of the CSC markers CD24, CD44, CD133, SOX2, OCT4 and NANOG.

To that end, cellular RNA was first extracted with Trizol (Invitrogen) reagent and quantified with NanoDrop 2000. 1 µg of RNA was used to carry out the retrotranscription process and obtain cDNA using SuperScript II Reverse Transcriptase (Invitrogen) with a universal reverse transcription primer. Real Time PCR was carried out using SYBR Green Supermix (iTaq Universal SYBR Green Supermix from Bio-Rad Laboratories, Hercules, CA). Gene expression data were normalized with GADPH. All quantitative RT-polymerase chain reaction (RT-PCR) assays were performed on an ABI 7900 (ABI) system, and the 2ΔΔCt method to calculate relative expression levels was applied.

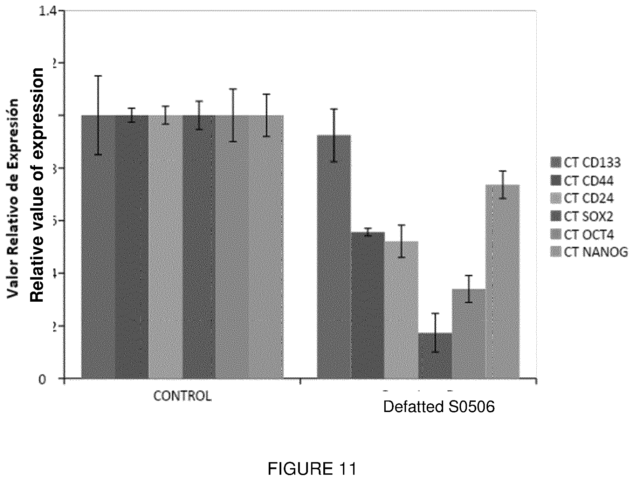

As observed in Fig. 11, the cultures treated with the extract from the defatted meal of *Solanum melongena* S0506 showed an expression of the selected markers significantly lower than that obtained in the controls. These results suggest that the extract, not only has an antitumor activity, but is capable of reducing the proportion of CSCs in the cultures. This result may have great clinical potential, since many of the current chemotherapy regimens not only do not affect CSCs but also select for this subpopulation, promoting early tumor reappearance and increasing its aggressiveness (Yujuan et al., 2018).

### Bibliographic references

Akihisa T, Takahashi A, Kikuchi T, Takagi M, Watanabe K, Fukatsu M, et al. The melanogenesis-inhibitory, anti-inflammatory, and chemopreventive effects of limonoids in n-hexane extract of Azadirachta indica A. Juss. (neem) seeds. Journal of Oil Science. 2011;60(2):53-9.

Amin A, Kucuk O, Khuri F, Shin D. Perspectives for Cancer Prevention with Natural Compounds. Journal of Clinical Oncology. 2009;27(16):2712-2725.

Bonta RK, Dietary Phenolic Acids and Flavonoids as Potential Anti-Cancer Agents: Current State of Art and Future Perspectives. Anticancer Agents Med Chem. 2019 (doi: 10.2174/1871520619666191019112712)

Bregni G, Akin Telli T, Camera S, Deleporte A, Moretti L, Bali AM, Liberale G, Holbrechts S, Hendlisz A, Sclafani F. Adjuvant chemotherapy for rectal cancer: Current evidence and recommendations for clinical practice. Cancer Treat Rev. 2020 Feb;83:101948.

Cohen E, Quistad GB, Casida JE. Cytotoxicity of nimbolide, epoxyazadiradione and other limonoids from neem insecticide. Life Sci. 1996;58(13):1075-1081.

Dewanto V, Wu X, Adom KK and Liu RH, Thermal processing enhances the nutritional value of tomatoes by increasing total antioxidant activity. J Agric Food Chem 50:3010-3014 (2002).

Duh PD, Tu YY and Yen GC, Antioxidant activity of the aqueous extract of harn jyur (Chrysanthemum morifolium Ramat). Lebensm-Wiss Technol 32:269-277 (1999).

Fekry MI, Ezzat SM, Salama MM, Alshehri OY, AI-Abd AM. Bioactive glycoalkaloids isolated from Solanum melongena fruit peels with potential anticancer properties against hepatocellular carcinoma cells. Scientific Reports. 2019; 11;9(1):1746.

Goyal S, Gupta N, Chatterjee S, Nimesh, S. Natural Plant Extracts as Potential Therapeutic Agents for the Treatment of Cancer. Current Topics in Medicinal Chemistry. 2017; 17(2):96-106.

Grada A, Otero-Vinas M, Prieto-Castrillo F, Obagi Z, Falanga V. Research Techniques Made Simple: Analysis of Collective Cell Migration Using the Wound Healing Assay. J Invest Dermatol. 2017;137(2):e11-e16. doi:10.1016/j.jid.2016.11.020

Hashimoto K, Kawagishi H, Nakayama T, Shimizu M. Effect of capsianoside, a diterpene glycoside, on tight-junctional permeability. Biochim Biophys Acta. 1997;1323(2):281-290.

Huang W, Cai Y, Zhang Y. Natural Phenolic Compounds From Medicinal Herbs and Dietary Plants: Potential Use for Cancer Prevention. Nutrition and Cancer. 2013;62(1):1-20.

Idrees M, Tejani M. Current Treatment Strategies for Elderly Patients with Metastatic Colon Cancer. Cureus. 2019 May 22;11(5):e4713.

Jayakumar K, Murugan K (2016) Solanum Alkaloids and their Pharmaceutical Roles: A Review. J Anal Pharm Res 3(6): 00075.

Jing P, Qian B, Zhao S, et al. Effect of glycosylation patterns of Chinese eggplant anthocyanins and other derivatives on antioxidant effectiveness in human colon cell lines. Food Chem. 2015;172: 183-189.

Kapravelou G, Martinez R, Andrade AM, López Chaves C, López-Jurado M, Aranda P, Arrebola F, Cañizares FJ, Galisteo M, Porres JM. Improvement of the antioxidant and hypolipidaemic effects of cowpea meals (Vigna unguiculata) by fermentation: results of in vitro and in vivo experiments. J Sci Food Agric 95(6):1207-1216 (2015).

Kintia PK; Shvets SA. Melongosides n o and p steroidal saponins from seeds of Solanum-Melongena. Phytochemistry. 1985; 24(7):1567-1570

Lee KR, Kozukue N, Han JS, Park JH, Chang EY, Baek EJ, Chang JS, Friedman M. Glycoalkaloids and metabolites inhibit the growth of human colon (HT29) and liver (HepG2) cancer cells. Journal of agricultural and food chemistry, 2004, 52(10):2832-2839.

Loree J, Kopetz S. Recent developments in the treatment of metastatic colorectal cancer. Therapeutic Advances in Medical Oncology. 2017;9(8):551-564.

Munro MJ, Wickremesekera SK, Peng L, Tan ST, Itinteang T. Cancer stem cells in colorectal cancer: a review. Journal of Clinical Pathology. 2018;71:110-116.

Nappi A, Berretta M, Romano C, Tafuto S, Cassata A, Casaretti R, Silvestro L, Divitiis C, Alessandrini L, Fiorica F, Ottaiano A, Nasti G. Metastatic Colorectal Cancer: Role of Target Therapies and Future Perspectives. Curr Cancer Drug Targets. 2018;18(5):421-429

Nishina A, Ebina K, Ukiya M, Fukatsu M, Koketsu M, Ninomiya M, et al. Dioscin Derived from Solanum melongena L. "Usukawamarunasu" Attenuates α-MSH-Induced Melanogenesis in B16 Murine Melanoma Cells via Downregulation of Phospho-CREB and MITF. Journal of Food Science. 2015; 80(10):H2354-9.

Ortiz R, Prados J, Melguizo C, et al. The cytotoxic activity of the phage E protein suppress the growth of murine B16 melanomas in vitro and in vivo. J Mol Med (Berl). 2009;87(9):899-911. doi:10.1007/s00109-009-0493-9

Reglero C, Reglero G. Precision Nutrition and Cancer Relapse Prevention: A Systematic Literature Review. Nutrients. 2019 Nov 16;11(11).

Rivas-Morales C, Oranday-Cárdenas MA, Verde-Star MJ. Investigación de plantas de importancia médica. Ed. IV. Universidad autónoma de nuevo León, México. 2016; ISBN: 978-84-94673-7-0.

Shen KH, Hung JH, Chang CW, Weng YT, Wu MJ, Chen PS, et al. Solasodine inhibits invasion of human lung cancer cell through downregulation of miR-21 and MMPs expression. Chemico-Biological Interactions. 2017; 25;268:129-135.

Thapliyal A, Krishen R, Chandra A. Overview of Cancer and Medicinal Herbs used for Cancer Therapy. Asian Journal of Pharmaceutics. 2018;12(1).

Ting H, Qi-Mei L, Xiao-Wei H, Fei H, Ming-Wei Z, Jian-Guo Jiang. Identification of bioactives from Astragalus chinensis L.f. and their antioxidant, anti-inflammatory and anti-proliferative effects. Journal of Food Science and Technology. 2017; 54(13):4315-4323.

Toshiyuki M, Kentarou K, Kiyofumi N, Hisashi M, Masayuki Y. Medicinal Foodstuffs. XXV.1) Hepatoprotective Principle and Structures of Ionone Glucoside, Phenethyl Glycoside, and Flavonol Oligoglycosides from Young Seedpods of Garden Peas, Pisum sativum L. Chemical & Pharmaceutical Bulletin. 2001; 49(8):1003-1008.

Wong F, Chai T, Xiao J. The influences of thermal processing on phytochemicals and possible routes to the discovery of new phytochemical conjugates. Critical Reviews in Food Science and Nutrition. 2018;1-6.

Wu X, Sooman L, Lennartsson J, Bergström S, Bergqvist M, Gullbo J, et al. Microtubule inhibition causes epidermal growth factor receptor inactivation in oesophageal cancer cells. International Journey of Oncology. 2013; 42(1):297-304.

Yin X, Yan L, Juan P, Hong-Liang Y, Yan S, Dong-Ying Z, Hai-Xue K et al. Melongenaterpenes A-L, Vetispirane-Type Sesquiterpenoids from the Roots of Solanum melongena. Journal of Natural Products. 2019; 82:3242-3248.

Yang BY, Yin X, Liu Y, Sun Y, Guan W, Zhou Y, et al. Terpenes and lignans from the roots of Solanum melongena L. Natural Product Research. 2020; 34(3):359-368.

Yu R, XUue W, Zhongguan L, Shuaishuai H, Haihui Z. et al. Induction of cell cycle arrest by increasing GTP-RhoA levels via Taxol-induced microtubule polymerization in renal cell carcinoma. Molecular Medicine Reports. 2017; 15:4273-4279.

Yujuan Z, Longzheng X, Heran W, Linda O, Min S, Qiang L, et al. Cancer stem cells in progression of colorectal cancer. Oncotarget. 2018; 9(70):33403-33415.

Zhao DY, Liu Y, Sun YP, Li XM, Xu ZP, Pan J, et al. Sesquiterpenoids with diverse carbon skeletons from the sepals of Solanum melongena L. Fitoterapia. 2020; 15;142:104517.

## Claims

1. A method for obtaining an ethanolic plant extract from the mature seed meal of *Solanum melongena,* which comprises the steps of:
a) grinding the seeds to obtain meal;
b) extracting the meal from step a) using a cold hydroalcoholic extraction solution at acid pH,
c) optionally, defatting the mature seeds by mechanical cold pressing before carrying out steps a) and b).

2. The method according to claim 1, wherein:
a) the seeds are ground to obtain meal with a particle size between 100 µm and 150 µm; and/or
b) the meal obtained in step a) is extracted with the extraction solution under the following operating conditions:
i. temperature equal to 4 ºC,
ii. in nitrogen atmosphere,
iii. the extraction solution is made up of ethanol, bidistilled water and 12N hydrochloric acid in proportions 50:50:0.2 by volume,
iv. pH equal to 2, and
v. the mixture of the meal and the extraction solution is kept under stirring for 30 minutes after having reached the conditions i to iv, wherein the extract is obtained by centrifuging the mixture of the extraction solution and the meal and collecting the supernatant.

3. The method according to claim 1 or 2, wherein step c) of defatting the mature seeds is carried out, at a temperature between 40 to 50 ºC and with an extraction rate of 2 to 3 kg of seeds/hour.

4. The method according to claim 2 or 3, wherein:
i) the precipitate resulting from centrifuging the mixture of meal and extraction solution is resuspended in extraction solution,
ii) the suspension obtained is kept under stirring again for 30 minutes under the conditions of step b) defined in claim 2,
iii) the suspension is subjected to centrifugation, collecting the supernatant, and
iv) the ethanolic extract results from mixing the supernatant obtained in iii) with the first supernatant obtained.

5. The method according to any of claims 1 to 4, which includes a final additional step in which the ethanol from the ethanolic extract obtained is partially or totally evaporated.

6. An ethanolic extract obtainable by the method of any of claims 1 to 5.

7. The ethanolic extract according to claim 6, which has a total polyphenol content ranging between 11.16 and 27.59 µg gallic acid equivalents/mg extract.

8. The ethanolic extract according to either of claims 6 or 7, wherein the total polyphenol content ranges between 11.16 and 18.41 µg gallic acid equivalents/mg extract and/or the reducing capacity ranges between 5.33 and 6.31 µg gallic acid equivalents/mg extract.

9. The ethanolic extract according to any of claims 6 to 8, wherein the total polyphenol content ranges between 12.06 and 27.59 µg gallic acid equivalents/mg extract.

10. The ethanolic extract according to any of claims 6 to 9, that comprises at least salannin and/or capsianoside II.

11. The ethanolic extract according to claim 10, that comprises salannin and capsianoside II and additionally, at least, myricomplanoside.

12. The ethanolic extract according to claim 10, that comprises salannin and capsianoside II and at least one additional compound selected from the group of kaempferol-3-sophorotrioside, myricomplanoside and aryllatose B, or combinations thereof.

13. The ethanolic extract according to claim 12, that comprises salannin and capsianoside II and additionally, at least, arylatose B.

14. The ethanolic extract according to any of claims 11 to 13, that comprises salannin and capsianoside II and additionally, at least, myricomplanoside and aryllatose B.

15. The ethanolic extract according to claim 12, that comprises salannin and capsianoside II and additionally, at least kaempferol 3-sophorotrioside and aryllatose B.

16. The ethanolic extract according to any of claims 11 to 15, that comprises salannin, capsianoside II, myricomplanoside, aryllatose B and kaempferol 3-sophorotrioside.

17. The ethanolic extract according to claim 16, wherein the total polyphenol content ranges between 11.16 and 18.41 µg gallic acid equivalents/mg extract and/or the reducing capacity ranges between 5.33 and 6.31 µg gallic acid equivalents/mg extract.

18. The ethanolic extract according to any of claims 6 to 17, which was obtained by the method of any of claims 1 to 5.

19. The ethanolic extract according to claim 18, wherein the final, partial or total evaporation of the ethanol as defined in claim 5 has been carried out.

20. The ethanolic extract according to claim 18 or 19, which is an ethanolic extract from defatted mature seeds wherein the step of defatting the mature seeds has been carried out by cold mechanical pressing before carrying out step a) of grinding the seeds and step b) of extracting the meal obtained.

21. The ethanolic extract according to claim 20, which was obtained by the method of claim 1 in which the step of defatting the mature seeds by cold mechanical pressing was carried out under the conditions of claim 3 before carrying out steps a) and b); step a) of grinding the seeds and step b) of extracting the meal obtained were carried out as defined in claim 2, and wherein a second extraction of the precipitate obtained after executing the method as defined in claim 2 is carried out following the conditions defined in claim 4.

22. A pharmaceutical composition that comprises an extract of any of claims 6 to 21 in its formulation.

23. The pharmaceutical composition according to claim 22, which is a combined pharmaceutical composition that additionally comprises at least one anticancer agent in addition to those present in the extract.

24. The pharmaceutical composition according to claim 22 or 23, that further comprises one or more pharmaceutically acceptable excipients or carriers.

25. The pharmaceutical composition according to any of claims 22 to 24, which includes an extract of any of claims 19 to 21 in its formulation.

26. An extract of any of claims 6 to 21 for use in the treatment of a type of cancer which is selected from the group of colorectal cancer, pancreatic cancer and glioblastoma.

27. The extract for use according to claim 26, wherein the type of cancer is selected from the group of colon adenocarcinoma, pancreatic adenocarcinoma and glioblastoma multiforme.

28. The extract for use according to claim 26 or 27, which is an extract from defatted mature seeds of claim 20 or 21.

29. The extract for use according to any of claims 26 to 28, which is an extract from mature seeds of any of claims 10 to 18.

30. The extract for use according to claim 29, for use in the treatment of colon adenocarcinoma.

31. The extract for use according to claim 29, for use in the treatment of chemotherapy-resistant colon adenocarcinoma.

32. The extract for use according to claim 29, for use in the treatment of pancreatic adenocarcinoma.

33. A pharmaceutical composition of any of claims 22 to 25, for use in the treatment of a type of cancer which is selected from the group of colorectal cancer, pancreatic cancer and glioblastoma.

34. The pharmaceutical composition for use according to claim 33, wherein the cancer is selected from the group of colon adenocarcinoma, pancreatic adenocarcinoma and glioblastoma multiforme.

35. The pharmaceutical composition for use according to claim 33 or 34, which is a composition of claim 25.

36. The pharmaceutical composition for use according to any of claims 34 or 35, wherein the cancer is colon adenocarcinoma.

37. The pharmaceutical composition for use according to claim 36, wherein the cancer is chemotherapy-resistant colon adenocarcinoma.

38. Use of the extract according to any of claims 6 to 21 as a functional ingredient to be incorporated into food supplements.

39. A food supplement that comprises the extract according to any of claims 6 to 21 as a functional ingredient.
